# EUROPÄISCHE PATENTSCHRIFT

(11) **EP 3 989 800 B1**
(45) Veröffentlichungstag und Bekanntmachung des Hinweises auf die Patenterteilung: **14.06.2023**
(21) Anmeldenummer: 20740169.6
(22) Anmeldetag: 18.06.2020
(51) Int. Cl.: A61B 5/00, A61N 1/00, A61B 5/291

(54) **ELEKTRODENANORDNUNG ZUR MESSUNG VON ELEKTRISCHEN SPANNUNGEN**
ELECTRODE ARRANGEMENT FOR MEASURING ELECTRIC VOLTAGES
AGENCEMENT D'ÉLECTRODES PERMETTANT DE MESURER DES TENSIONS ÉLECTRIQUES

(30) Priorität: 26.06.2019 AT 505722019
(43) Veröffentlichungstag der Anmeldung: 04.05.2022
(73) Patentinhaber: Guger, Christoph, 4521 Schiedlberg (AT); Edlinger, Günter, 8055 Seiersberg-Pirka (AT)
(72) Erfinder: Guger, Christoph, 4521 Schiedlberg (AT); Edlinger, Günter, 8055 Seiersberg-Pirka (AT)
(74) Vertreter: Wildhack & Jellinek
(86) Internationale Anmeldenummer: PCT/AT2020/060247
(87) Internationale Veröffentlichungsnummer: WO 2020/257836

(56) Entgegenhaltungen:
- WO-A1-98/02090
- WO-A2-2009/139911
- WO-A2-2012/088398
- JP-B2- 5 612 051
- US-A1- 2016 296 135
- US-A1- 2017 086 686
- US-A1- 2017 128 015
- US-A1- 2020 138 313
- US-B2- 9 731 108

## Beschreibung

Die Erfindung betrifft eine Elektrodenanordnung zur Messung von elektrischen Spannungen und Strömen im menschlichen Körper sowie zur Elektro-Stimulation des menschlichen Körpers gemäß Patentanspruch 1.

Aus dem Stand der Technik sind verschiedenste Elektrodenanordnungen zur Vermessung von Hirnströmen oder der Herzaktivität besonders aus dem medizinischen Bereich bekannt. Derartige bekannte Elektrodenanordnungen umfassen beispielsweise Trockenelektroden oder Elektroden, die beispielsweise mit Hilfe von elektrisch leitfähigem Gel oder elektrisch leitfähigen Flüssigkeiten einen Kontakt zur Körperoberfläche eines Probanden herstellen, sodass elektrische Signale im Inneren des Körpers des Probanden detektiert werden können.

Aus der WO 98/02090 ist eine Elektrodenanordnung bekannt, die ein rasterförmiges Elektroden-Segmente auf einem flexiblen Film sowie eine flexible Schaumschicht mit Löchern für elektrisch leitfähiges Gel umfasst. Aus der US 9731108 B2 ist eine Elektrodenanordnung bekannt, die ein Elektroden-Array auf einem Träger mit Schlitzen, auf dem Hydrogel aufgebracht ist, sowie ein Rückhaltenetz zur Abdeckung der Elektroden aufweist.

Bei allen Elektrodenanordnungen sind für ein einwandfreies Messergebnis jedoch eine korrekte Positionierung der Elektroden, eine gute Fixierung bzw. ein guter Halt auf dem zu vermessenden Körperteil des Probanden, sowie ein einwandfreier elektrischer Kontakt der Elektroden zur Körperoberfläche erforderlich. Ein derartiger einwandfreier Kontakt bzw. eine gute Fixierung sind insbesondere an gekrümmten Körperoberflächen wie beispielsweise dem Kopf eines Probanden schwierig herzustellen. Weiters ist es insbesondere im Bereich der Elektroenzephalographie wünschenswert, eine hohe Elektrodendichte pro Flächeneinheit der zu untersuchenden Körperoberfläche zur Verfügung zu haben, um eine möglichst gute räumliche Auflösung der EEG-Messdaten gewährleisten zu können, um beispielsweise Aktivitäten in bestimmten Gehirnregionen verorten zu können. Da bei bekannten Elektrodenanordnungen die einzelnen Elektroden jedoch häufig einzeln platziert werden müssen, ist die Positionierung einer großen Anzahl an Elektroden auf der Körperoberfläche des Probanden mit einem hohen Zeitaufwand verbunden.

Aufgabe der Erfindung ist es daher, eine Elektrodenanordnung zur Verfügung zu stellen, die diesbezüglich Abhilfe schafft und eine einfache Platzierung einer Vielzahl von Elektroden auch an gekrümmten Körperoberflächen ermöglicht und gleichzeitig elektrisch leitfähiges Gel zurückhält.

Die Erfindung löst diese Aufgabe mit einer Elektrodenanordnung zur Messung von elektrischen Spannungen und Strömen im menschlichen Körper sowie zur Elektrostimulation des menschlichen Körpers gemäß Patentanspruch 1. Dabei ist vorgesehen, dass die Elektrodenanordnung
- ein Elektroden-Array mit einem flexiblen Träger, wobei eine Anzahl von Elektroden rasterförmig auf derselben Seite des Trägers angeordnet sind und wobei vom äußeren Rand des Trägers her Schlitze zwischen den einzelnen Elektroden angeordnet sind, und
- eine elastische Zwischenschicht, die für jede der Elektroden jeweils eine Ausnehmung aufweist, wobei durch jede Ausnehmung jeweils ein Hohlraum zur Verfüllung mit elektrisch leitfähigem Gel vorgegeben ist, umfasst.

Erfindungsgemäß ist dabei eine zwischen der elastischen Zwischenschicht und den Elektroden des Elektroden-Arrays angeordnete Rückhalteschicht vorgesehen,
- wobei die Rückhalteschicht eine kleinere Querschnittsfläche aufweist, als die elastische Zwischenschicht, und
- wobei die Rückhalteschicht Kontaktöffnungen aufweist,
   wobei die Kontaktöffnungen eine kleinere Querschnittsfläche aufweisen, als die Ausnehmungen der Zwischenschicht und
   wobei die Kontaktöffnungen, insbesondere in einem an der Körperoberfläche eines Probanden angeordneten Zustand der Elektrodenanordnung, derart mit den Ausnehmungen korrespondieren, dass eine elektrisch leitfähige Verbindung zwischen den Elektroden und den Hohlräumen, insbesondere zwischen den Elektroden und einem in den Hohlräumen vorhandenem elektrisch leitfähigen Gel, herstellbar ist.

Eine derartig ausgestaltete Elektrodenanordnung ermöglicht es vorteilhafterweise, eine Vielzahl von Elektroden gleichzeitig in einem Arbeitsschritt an der Körperoberfläche eines Probanden anzubringen, da diese in Form eines Elektroden-Arrays zusammenhängend angeordnet sind. Ein einwandfreier leitfähiger Kontakt zur Körperoberfläche des Probanden ist vorteilhafterweise dadurch sichergestellt, dass in den Ausnehmungen für jede einzelne Elektrode ein Gel zur Herstellung einer leitfähigen Verbindung zur Körperoberfläche vorhanden ist. Durch die Schlitze im Träger, die zwischen die einzelnen Elektroden eingreifen, ist eine Anpassung des Elektroden-Arrays bzw. ein Anlegen der Elektrodenanordnung auch an gekrümmte Körperoberflächen problemlos möglich.

Eine derartige Elektrodenanordnung, ist auch leicht zu reinigen und gewährleistet gleichzeitig einen besonders guten elektrisch leitfähigen Kontakt zwischen den Elektroden und einer Körperoberfläche des menschlichen Körpers.

Um eine besonders rasche Montage der Elektrodenanordnung an der Körperoberfläche eines Probanden zu gewährleisten, kann vorgesehen sein, dass die elastische Zwischenschicht eine erste klebende Oberfläche zur Anordnung an der Körperoberfläche eines Probanden und/oder eine zweite klebende Oberfläche zur Anordnung am Träger des Elektroden-Arrays aufweist.

Eine besonders gute Anpassung der elastischen Zwischenschicht an verschiedenst geformte Körperoberflächen bei gleichzeitig geringen Herstellungskosten kann erzielt werden, wenn die elastische Zwischenschicht aus einem flexiblen elektrisch isolierendem Material, insbesondere einem geschäumten Kunststoff, gebildet ist.

Zur Verringerung des Kontaktwiderstands und der Kontaktspannung kann vorgesehen sein, dass die Elektroden des Elektroden-Arrays hochleitfähig sind, und insbesondere aus Gold, Platin, elektrisch leitfähigem Polymer oder einem anderen Elektrodenmaterial bestehen.

Ein besonders einfach herzustellender Träger auch für eine große Anzahl von Elektroden kann bereitgestellt werden, wenn der Träger des Elektroden-Arrays als flexible Leiterplatte, insbesondere als FlexPrint, ausgebildet ist.

Ein besonders vielfältiger Einsatz der Elektrodenanordnung zur Messung von elektrischen Spannungen und Strömen im menschlichen Körper, aber auch zur Elektrostimulation des menschlichen Körpers kann erzielt werden, wenn das Elektroden-Array zur Anordnung an einem Fuß und/oder am Brustkorb und/oder am Herzen ausgebildet ist.

Eine besonders zuverlässige Fixierung der Elektrodenanordnung an verschiedensten Körperoberflächen kann erzielt werden, wenn die Schlitze zwischen den einzelnen Elektroden des Elektroden-Arrays derart ausgebildet sind, dass das Elektroden-Array an einer gekrümmten Körperoberfläche, insbesondere am Kopf eines Probanden, anordenbar ist.

Ein besonders zuverlässiges Messergebnis kann erzielt werden, wenn eine auf dem Träger angeordnete Verstärkeranordnung vorgesehen ist, wobei die Elektroden an die Verstärkeranordnung angeschlossen sind, und wobei insbesondere vorgesehen ist, dass die Verstärkeranordnung in einem Abstand von kleiner als 100 mm, insbesondere im Bereich unter 10 mm, zur nächstgelegenen Elektrode des Elektroden-Arrays angeordnet ist. Durch die Anordnung der Verstärkeranordnung auf dem Träger der Elektrodeanordnung kann sichergestellt werden, dass keine störenden Einflüsse das Messergebnis verfälschen, da insbesondere bei kleinen Elektroden eine große Impedanz auftreten kann, die das Messergebnis verfälschen kann.

Ein besonders einfacher Wechsel zwischen einer Messung von elektrischen Strömen oder Spannungen des menschlichen Körpers zu einer Elektrostimulation des menschlichen Körpers kann gewährleistet werden, wenn eine mit dem Elektroden-Array verbundene Switching-Unit, wobei die Switching-Unit dazu ausgebildet ist, die zwischen einer Elektro-Stimulation des menschlichen Körpers über die Elektroden und einer Messung von elektrischen Spannungen und Strömen im menschlichen Körper mittels der Elektroden umzuschalten.

Eine besonders rasch anzubringende Elektrodenanordnung kann bereitgestellt werden, wenn die Hohlräume, insbesondere in einem an der Körperoberfläche eines Probanden angeordneten Zustand der Elektrodenanordnung, mit elektrisch leitfähigem Gel gefüllt sind.

Eine besonders materialsparende Ausgestaltungsvariante der Zwischenschicht kann bereitgestellt werden, wenn die elastische Zwischenschicht, insbesondere in einem an der Körperoberfläche eines Probanden angeordneten Zustand der Elektrodenanordnung, vom Träger des Elektroden-Arrays abgedeckt ist.

Eine Elektrodenanordnung kann folgendermaßen an eine Körperoberfläche des menschlichen Körpers angelegt werden:
a) die zweite klebende Oberfläche der Zwischenschicht wird derart auf dem Träger des Elektroden-Arrays angeordnet, dass die Elektroden mit den Ausnehmungen in der Zwischenschicht korrespondieren,
b) elektrisch leitfähiges Gel wird derart auf die vom Träger des Elektroden-Arrays abgewandte Seite der Zwischenschicht aufgetragen, dass die von den Ausnehmungen vorgegebenen Hohlräume vollständig gefüllt sind, wobei insbesondere vorgesehen ist, dass überschüssiges Gel entfernt wird, und
c) die erste klebende Oberfläche der Zwischenschicht wird an einer Körperoberfläche des menschlichen Körpers angeordnet.

Dies kann auch in der umgekehrten Reihenfolge durchgeführt werden.

Weitere Vorteile und Ausgestaltungen ergeben sich aus der Beschreibung und den beiliegenden Zeichnungen.

Besonders vorteilhafte, aber nicht einschränkend zu verstehende Ausführungsbeispiele der Erfindung werden im Folgenden anhand der beiliegenden Zeichnungen schematisch dargestellt und unter Bezugnahme auf die Zeichnungen beispielhaft beschrieben.

Im Folgenden zeigen:
Fig. 1 ein erstes Ausführungsbeispiel einer Elektrodenanordnung,
Fig. 2 die Elektrodenanordnung aus Fig. 1 im am Kopf eines Probanden angeordneten Zustand,
Fig. 3 bis Fig. 7 weitere Ausführungsbeispiele von Elektroden-Arrays.

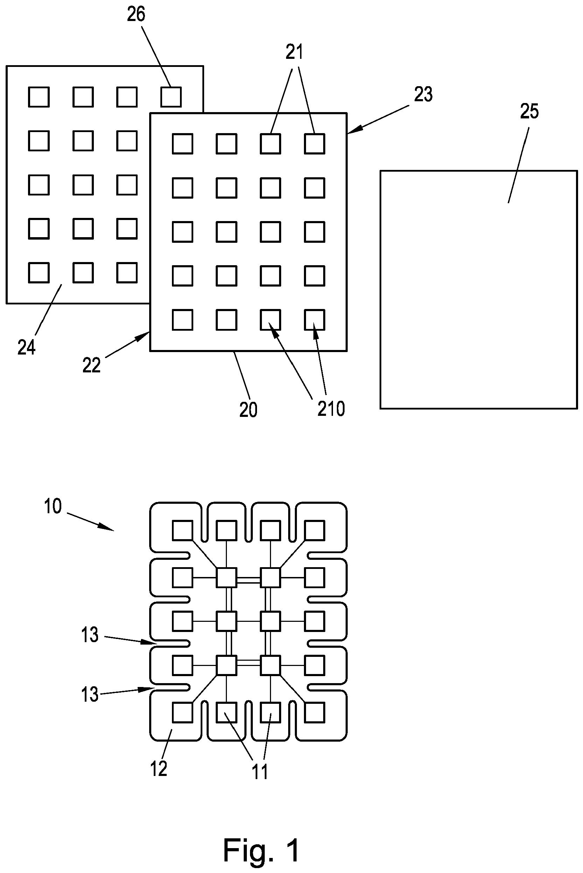

Fig. 1 zeigt ein erstes Ausführungsbeispiel einer Elektrodenanordnung 100. Die Elektrodenanordnung 100 umfasst ein Elektroden-Array 10 mit einem flexiblen, d.h. elastischen, biegsamen, Träger 12, wobei die Elektroden 11 des Elektroden-Arrays 10 auf dem flexiblen Träger 12 angeordnet sind. Die Elektroden 11 sind im ersten Ausführungsbeispiel rechteckig ausgebildet und rasterförmig auf der selben Seite des Trägers 12 angeordnet, sowie äquidistant verteilt. Eine derartige Anordnung der Elektroden 11 ist jedoch keinesfalls zwingend für die Funktionsweise der Erfindung und auch andere Anordnungen z.B. in Form von konzentrischen Kreisen oder mit variierendem Abstand der Elektroden 11 zueinander sind möglich. Optional können die Elektroden 11 auch z.B. eine kreisförmige Querschnittsfläche aufweisen.

Im ersten Ausführungsbeispiel in Fig. 1 handelt es sich beim Träger 12 um eine flexible Leiterplatte, die auch als Flex Print bekannt ist. Bei einer derartigen flexiblen Leiterplatte handelt es sich um ein Substrat beispielsweise aus Polyimid mit einer Dicke von z.B. kleiner als 200 µm, das in beliebige Form und Größe geschnitten werden kann. Die Leiterbahnen können beispielsweise aus Kupfer hergestellt sein, eine Dicke von z.B. 28 µm aufweisen und auch an beiden Seiten des Trägers 12 vorhanden sein.

Die auf dem Träger 12 angeordneten Elektroden 11 sind im gezeigten Ausführungsbeispiel in Fig. 1 hoch leitfähig und aus Gold hergestellt, können aber auch aus Platin oder leitfähigem Polymer oder einem anderen bekannten Elektrodenmaterial gebildet sein. Weiters alternativ dazu können die Elektroden 11 beispielsweise aus Nickel überzogen mit einer Goldschicht von beispielsweise 0,03 µm gebildet sein. Das Substrat des Trägers 12 kann beispielsweise auch darauf aufgebrachte Deckschichten wie Epoxidharz oder eine zusätzliche Polyimid-Schicht aufweisen, die in den Bereichen zwischen den Elektroden 11 aufgebracht sind. Die maximale Dicke des Elektroden-Arrays 10 mit dem flexiblen Träger 12 und den Elektroden 11 kann beispielsweise weniger als 1 mm betragen.

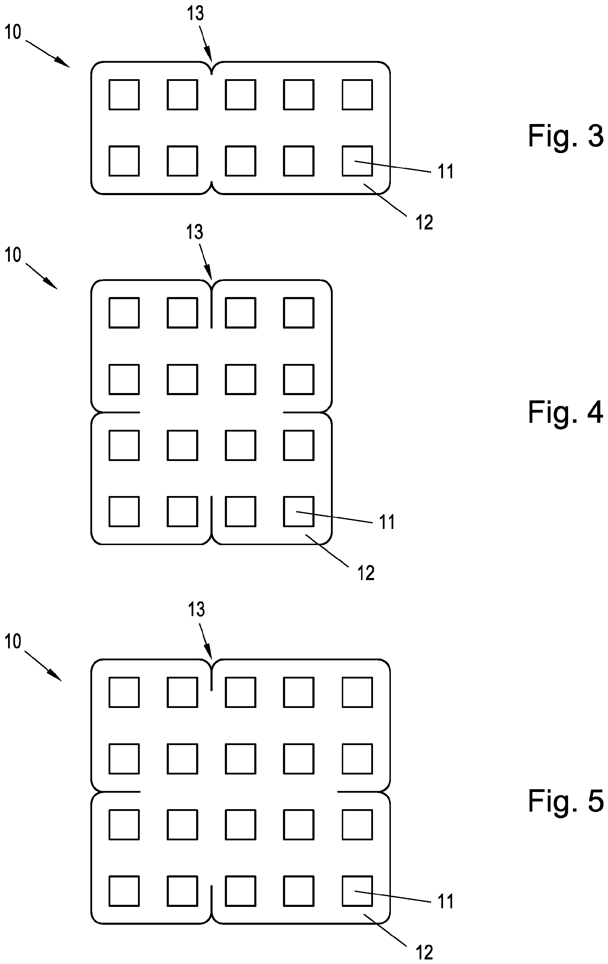

Im Ausführungsbeispiel in Fig. 1 sind 20 Elektroden 11 in fünf Zeilen mit jeweils vier Spalten angeordnet. Die Elektroden 11 haben im ersten Ausführungsbeispiel in Fig. 1 eine Größe von 2 mal 2 mm und sind äquidistant auf einem Träger 12 der Größe 5 mal 5 cm angeordnet. Die Größe des Trägers 12, aber auch die Anzahl an Elektroden 11, die auf dem Träger 12 angeordnet sind, kann jedoch auch anders gewählt sein. Ausführungsbeispiele für derartige Elektroden-Arrays 10 mit einer variierenden Anzahl an Elektroden 11 sind in Fig. 3 bis Fig. 7 dargestellt, worauf im Folgenden noch näher eingegangen wird.

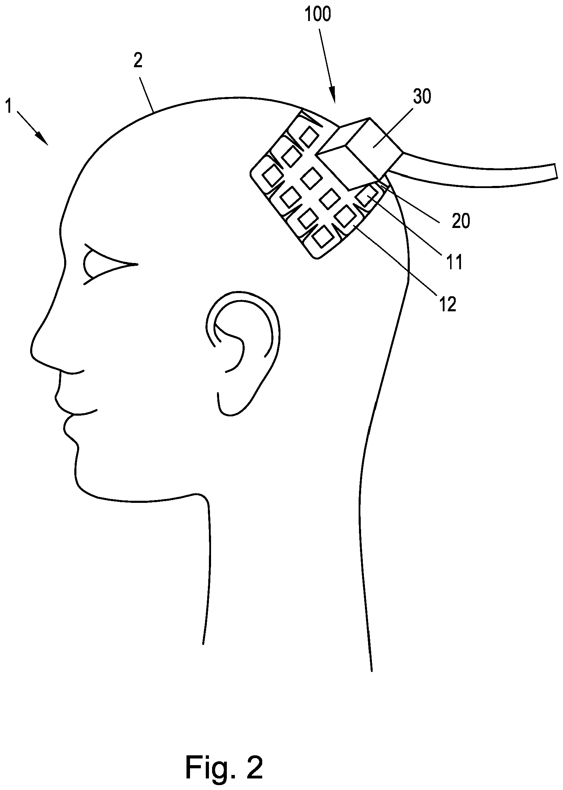

Zwischen den rasterförmig auf den Träger 12 angeordneten Elektroden 11 sind vom äußeren Rand des Trägers 12 her Schlitze 13 zwischen den einzelnen Elektroden angeordnet. Im gezeigten Ausführungsbeispiel reichen die Schlitze 13 durch die vom Rand des Trägers 12 her betrachtet äußerste Reihe an Elektroden 11 hindurch. Dies ist jedoch keinesfalls zwingend erforderlich und die Schlitze 13 können kürzer gewählt sein, aber, je nach Größe des Trägers 12, auch durch beispielsweise zwei oder mehr Elektrodenreihen hindurchreichen. Diese Schlitze 13 stellen vorteilhafterweise sicher, dass sich der als flexible Leiterplatte ausgebildete Träger 12 an die Körperoberfläche eines Probanden 1 anpassen kann, sodass eine derartige Elektrodenanordnung 100 auch an gekrümmte Körperoberflächen angelegt werden kann, wie dies für den Kopf 2 eines Probanden 1 in Fig. 2 dargestellt ist.

Eine erfindungsgemäße Elektrodenanordnung 100 kann jedoch auch an einem Fuß, am Brustkorb oder am Herzen angeordnet werden. Die Dimensionen des Trägers 10 und der Zwischensicht 20, sowie die Länge der Schlitze 13 sind in diesem Fall für die Anordnung an der jeweiligen Körperoberfläche angepasst.

Eine erfindungsgemäße Elektrodenanordnung 100 umfasst, wie in Fig. 1, eine elastische Zwischenschicht 20, die für jede der Elektroden 11 des Elektroden-Arrays 10 jeweils eine Ausnehmung 21 aufweist. Die einzelnen Ausnehmungen 21 der elastischen Zwischenschicht 20 sind mit Gel zur Herstellung einer leitfähigen Verbindung zur Körperoberfläche des menschlichen Körpers gefüllt oder damit befüllbar.

Ist die Elektrodenanordnung 100, wie in Fig. 2 dargestellt, an der Körperoberfläche eines Probanden 1 angeordnet, so deckt der Träger 12 des Elektroden-Arrays 10 die elastische Zwischenschicht 20 ab. Im ersten Ausführungsbeispiel ist die elastische Zwischenschicht 20 aus einem flexiblen, geschäumten und elektrisch isolierendem Material, in Fig. 1 ist dies ein Kunststoff, gebildet und weist eine erste klebende Oberfläche 22 zur Anordnung an der Körperoberfläche eins Probanden 1 und eine zweite klebende Oberfläche 23 zur Anordnung am Träger 12 des Elektroden-Arrays 10 auf.

Bei der Zwischenschicht 20 kann es sich beispielsweise um ein hautfreundliches doppelseitig klebendes Klebeband handeln, das beispielsweise eine Dicke von z.B. ca. 1700 µm aufweisen kann. Die Ausnehmungen 21 für die Elektroden 11 können dabei beispielsweise durch Stanzen oder Laserschneiden in der Zwischenschicht 20 hergestellt werden. Die Ausnehmungen 21 können dabei dieselbe Größe wie die Elektroden 11 aufweisen oder leicht vergrößert sein, sodass die Kontaktfläche zwischen dem leitfähigen Gel und der Körperoberfläche bzw. der Haut des Probanden 1 maximiert ist und gleichzeitig die Distanz zwischen den Ausnehmungen 21 groß genug ist, um einen elektrischen Kurzschluss zu vermeiden.

Von den Ausnehmungen 21 werden Hohlräume 210 vorgegebenen, die mit elektrisch leitfähigem Gel befüllt werden können, um einen möglichst guten elektrischen Kontakt zur Körperoberfläche herzustellen. Das Volumen eines derartigen Hohlraums 210 ist durch die Querschnittsfläche der jeweiligen Ausnehmung 21 und die Dicke der Zwischenschicht 20 definiert.

Im Folgenden wird nun ein Verfahren zum Anlegen einer Elektrodenanordnung 100 an der Körperoberfläche eines Probanden 1 beschrieben, welches nicht unter die Patentansprüche fällt. Wie in Fig. 1 ersichtlich ist, weist die Zwischenschicht 20 im ersten Ausführungsbeispiel in einem Ausgangszustand, d.h. vor der Anordnung an der Körperoberfläche des Probanden 1, zunächst eine erste Abdeckschicht 24 auf, die Öffnungen 26 aufweist, die mit den Ausnehmungen 21 in der Zwischenschicht 20 korrespondieren. Die erste Abdeckschicht 24 deckt die erste klebende Oberfläche 22 der Zwischenschicht 20 ab, mit der die Zwischenschicht 20 an der Körperoberfläche eines Probanden 1 angeordnet wird, beispielsweise am Kopf 2 des Probanden 1, wie dies in Fig. 2 dargestellt ist. Durch die erste Abdeckschicht 24 ist die erste klebende Oberfläche 22 vor Verschmutzungen, die die Haftwirkung beeinträchtigen könnten, geschützt.

Wie in Fig. 1 weiters ersichtlich ist, weist die elastische Zwischenschicht 20 im ersten Ausführungsbeispiel im Ausgangszustand eine zweite, geschlossene Abdeckschicht 25 auf, die die zweite klebende Oberfläche 23 abdeckt, mit der die Zwischenschicht 20 am Träger 12 des Elektroden-Arrays 10 angeordnet wird. Dies dient zum Schutz der zweiten klebenden Oberfläche 23 vor Verschmutzung.

Bei einem Verfahren zum Anbringen einer Elektrodenanordnung 100 an einer Körperoberfläche des menschlichen Körpers (welches nicht unter die Patentansprüche fällt) wird zunächst die zweite klebende Oberfläche 23 der Zwischenschicht 20 derart auf dem Träger 12 des Elektroden-Arrays 10 angeordnet, dass die Elektroden 11 mit den Ausnehmungen 21 in der Zwischenschicht 20 korrespondieren, sodass elektrische Spannungen oder Ströme durch die Ausnehmungen 21 hindurch messbar sind. Ist die zweite klebende Oberfläche 23 der Zwischenschicht 20 von einer zweiten Abdeckschicht 25 bedeckt, wird diese zunächst abgezogen, um die Verbindung zwischen der Zwischenschicht 20 und dem Träger 12 des Elektroden-Arrays 10 herzustellen.

Danach wird elektrisch leitfähiges Gel derart auf die vom Träger 10 des Elektroden-Arrays 10 abgewandte Seite der Zwischenschicht 20 aufgetragen, sodass die von den Ausnehmungen 21 vorgegebenen Hohlräume 210 vollständig gefüllt sind und anschließend wird überschüssiges Gel beispielsweise mit einer Karte abgezogen.

Anschließend wird, sofern sie vorhanden ist, die erste Abdeckschicht 24, die die erste klebende Oberfläche 22 der Zwischenschicht 20 abdeckt und in der die Öffnungen 26, die mit den Ausnehmungen 21 in der Zwischenschicht 20 korrespondieren, angeordnet sind, abgezogen. Dies stellt sicher, dass das elektrisch leitfähige Gel ausschließlich in den Ausnehmungen 21 vorhanden ist, sodass elektrische Kurzschlüsse zwischen den Elektroden 11 vermieden werden.

Danach wird die Zwischenschicht 20 mit der ersten klebenden Oberfläche 22 an der Körperoberfläche des Probanden 1 angeordnet, wie dies in Fig. 2 dargestellt ist. Um eine möglichst gute Fixierung und einen guten Kontakt der Elektroden 11 zur Körperoberfläche sicherzustellen, ist es vorteilhaft, wenn die Haut des Probanden 1 frei von Haaren und losen Hautschuppen ist.

Eine weitergehende Vorbereitung der Haut beispielsweise mit abrasivem Gel oder Alkohol, wie dies für aus dem Stand der Technik bekannte Elektrodenanordnungen erforderlich ist, ist bei einer erfindungsgemäßen Elektrodenanordnung 100 vorteilhafterweise nicht erforderlich.

Auf diese Weise kann eine rasche Montage einer großen Anzahl von Elektroden 11 erzielt werden, da diese gemeinsam auf dem Träger 10 angeordnet sind und auch das elektrisch leitfähige Gel nicht in jede Ausnehmung 21 separat eingebracht werden braucht.

Ein Verfahren zum Anlegen einer Elektrodenanordnung (welches nicht unter die Patentansprüche fällt) kann vorteilhafterweise auch in der umgekehrten Reihenfolge durchgeführt werden, d.h. die Zwischenschicht 20 wird zuerst an der Körperoberfläche des menschlichen Körpers platziert und anschließend wird der Träger 12 des Elektroden-Arrays 10 mit der Zwischenschicht 20 verbunden.

Durch die Platzierung einer großen Anzahl an Elektroden 11 kann eine große Menge an EEG-Daten gewonnen werden, ohne dass z.B. das Gehirn des Probanden 1 geöffnet werden muss, sodass es beispielsweise auch möglich ist, Dipole im Gehirn des Probanden 1 zu ermitteln bzw. genau zu lokalisieren. Dies ist beispielsweise besonders hilfreich, um Epilepsie-Marker im Gehirn zu ermitteln und derart Gehirnareale für eine mögliche operative Behandlung zu lokalisieren.

Sind eine erste Abdeckschicht 24 und eine zweite Abdeckschicht 25 im Ausgangszustand an der Zwischenschicht 20 angeordnet, so kann das elektrisch leitfähige Gel auch bereits beim Herstellungsprozess der Zwischenschicht 20 in die Ausnehmungen 21 bzw. die Hohlräume 210 eingebracht und mit den Abdeckschichten 24, 25 eingeschlossen werden, sodass ein nachträgliches Einbringen nicht erforderlich ist. Über der ersten Abdeckschicht 24 kann in diesem Fall eine weitere Abdeckschicht angeordnet sein, die verhindert, dass das Gel aus den Ausnehmungen 21 bzw. aus den Hohlräumen 210 ausläuft.

Nach der Verwendung kann die Zwischenschicht 20 vom Elektroden-Array 10 bzw. vom flexiblen Träger 12 abgelöst werden und das Elektroden-Array 10 gereinigt werden, sodass es anschließend wiederverwendbar ist. Im Ausführungsbeispiel in Fig. 2 umfasst die Elektrodenanordnung 100 vorteilhafterweise eine Verstärkeranordnung 30, an die die Elektroden 11 angeschlossen sind. Die Verstärkeranordnung 30 ist auf dem Träger 12 angeordnet und beispielsweise in einem Abstand von kleiner als 100 mm, insbesondere kleiner als 10 mm, zur nächstgelegenen Elektrode 11 des Elektroden-Arrays 10 angeordnet, wie dies in Fig. 2 dargestellt ist. Im vorliegenden Ausführungsbeispiel beträgt dieser Abstand ca. 5 mm. Da bei Elektroden 11 mit einer kleinen Fläche die Impedanz vergleichsweise hoch ist und dies den Messwert beeinträchtigen kann, ist eine Verstärkeranordnung 30, die auf dem Träger 12 angeordnet ist, besonders vorteilhaft, da auf diese Weise derartige Störungen vermieden werden.

Die zuvor angeführte Beschreibung von Figuren, Ausführungsbeispielen bzw. Verfahrensschritten gilt auch für eine erfindungsgemäße Elektrodenanordnung 100. Eine erfindungsgemäße Elektrodenanordnung 100 umfasst zusätzlich eine nicht dargestellte, zwischen der elastischen Zwischenschicht 20 und den Elektroden 11 des Elektroden-Arrays 10 angeordnete Rückhalteschicht. Eine derartige Rückhalteschicht weist eine kleinere Querschnittsfläche auf, als die elastische Zwischenschicht 20. Auf diese Weise ist sichergestellt, dass z.B. ein ausreichend großer Bereich der zweiten klebenden Oberfläche 23 frei bleibt und die Zwischenschicht 20 zuverlässig mit dem Träger 12 des Elektroden-Arrays 10 verbunden bzw. verklebt werden kann.

Die Rückhalteschicht weist Kontaktöffnungen auf, die eine kleinere Querschnittsfläche aufweisen, als die Ausnehmungen 21 der Zwischenschicht 20, sodass einerseits elektrisch leitfähiges Gel, das sich in den Hohlräumen 210 befindet, zurückgehalten wird und nicht ausläuft.

Andererseits ist durch die Kontaktöffnungen hindurch eine elektrisch leitfähige Verbindung zwischen den Elektroden 11 und den Hohlräumen 210 bzw. dem in den Hohlräumen 210 vorhandenem elektrisch leitfähigen Gel und somit zur Körperoberfläche des Probanden 1 herstellbar, wenn die Rückhalteschicht derart zwischen der Zwischenschicht 20 und dem Elektroden-Array 10 angeordnet ist, dass die Kontaktöffnungen mit den Ausnehmungen 21 der Zwischenschicht 20 korrespondieren.

Unter "korrespondieren" ist in diesem Zusammenhang zu verstechen, dass jeweils ein Teil der Querschnittsfläche einer Ausnehmung 21 mit der Querschnittsfläche einer Kontaktöffnung zur Deckung kommt, sodass z.B. elektrisch leitfähiges Gel aus dem jeweiligen Hohlraum 210 durch die Rückhalteschicht hindurch die jeweilige Elektrode 11 kontaktiert. Dies kann z.B. erzielt werden, wenn jeweils der Mittelpunkt der Querschnittsflächen der Kontaktöffnung und der Ausnehmung übereinstimmen.

Weiters optional kann die elastische Zwischenschicht 20 auch ohne klebende Oberflächen 22, 23 oder mit nur einer klebenden Oberfläche ausgebildet sein. In diesem Fall kann die Elektrodenanordnung 100 beispielsweise mit selbstklebendem medizinischen Verbandmaterial an der Körperoberfläche eines Probanden 1 fixiert werden. Das Verbandmaterial wird beispielsweise auf dem Träger 12 fixiert oder über den Träger 12 hinweg gespannt und.mit der betreffenden Körperoberfläche verklebt, sodass auch die Zwischenschicht 20 sicher an der Körperoberfläche fixiert ist.

Optional kann eine erfindungsgemäße Elektrodenanordnung 100 auch eine Switching-Unit umfassen, die zum Umschalten zwischen einer Messung bzw. einer Stimulation mittels der Elektroden 11 dient. Auf diese Weise kann entweder eine Messung von elektrischen Spannungen und Strömen im menschlichen Körper erfolgen oder eine Stimulation der betreffenden Körperoberfläche erfolgen.

In Fig. 3 bis Fig. 7 sind weitere Ausführungsbeispiele mit möglichen Konfigurationen eines Elektroden-Arrays 10 dargestellt.

Fig. 3 zeigt eine Elektrodenanordnung 100, bei der zehn quadratische Elektroden 11 auf einem Träger 12 in zwei Zeilen zu jeweils fünf Elektroden 11 angeordnet sind. Schlitze 13 sind in jeder Reihe zwischen der zweiten und der dritten Elektrode angeordnet.

Fig. 4 zeigt eine Elektrodenanordnung 100, bei der sechzehn quadratische Elektroden 11 auf einem Träger 12 in vier Zeilen zu jeweils vier Elektroden 11 angeordnet sind. Schlitze 13 sind am Rand des Trägers 10 zwischen der zweiten und der dritten Elektrode angeordnet.

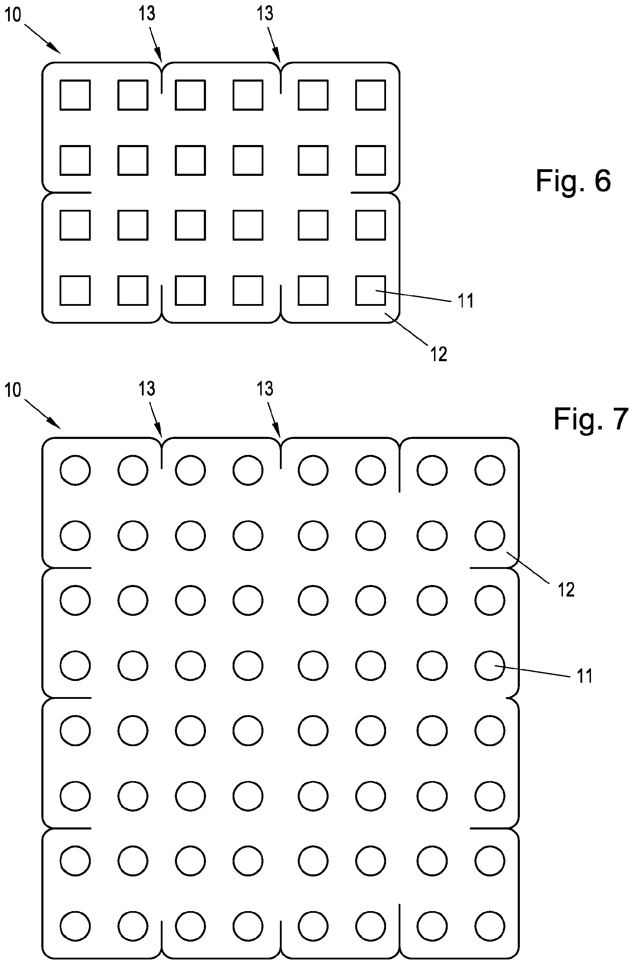

Fig. 5 und 6 zeigen ähnliche Elektrodenanordnungen 100 mit zwanzig quadratischen Elektroden 11 auf einem Träger 12 in vier Zeilen zu jeweils fünf Elektroden 11 und zweiunddreißig quadratischen Elektroden 11 auf einem Träger 12 in vier Zeilen zu jeweils acht Elektroden 11.

Beispielsweise können, wie in Fig. 7 dargestellt, auch 64 Elektroden 11 mit kreisförmigem Querschnitt auf einem 8 mal 8 cm großen Träger 12 rasterförmig und äquidistant verteilt angeordnet sein. In diesem Fall können auch die Ausnehmungen 21 in der Zwischenschicht 20 und gegebenenfalls die Öffnungen 26 in der ersten Abdeckschicht 24 einen kreisförmigen Querschnitt aufweisen. In Fig. 7 sind an jeder Kante des Trägers 12 drei Schlitze 13, d.h. jeweils nach zwei Elektroden 11, angeordnet, die jeweils bis die durch vom Rand des Trägers 12 her betrachtet äußerste Reihe an Elektroden 11 hindurch reichen. Die Elektroden 11 haben in Fig. 7 einen Durchmesser von 4 mm und der Abstand vom Zentrum einer Elektrode 11 zum Zentrum einer jeweils benachbarten Elektrode 11 beträgt 10 mm.

## Patentansprüche

1. Elektrodenanordnung (100) zur Messung von elektrischen Spannungen und Strömen im menschlichen Körper sowie zur Elektro-Stimulation des menschlichen Körpers umfassend
- ein Elektroden-Array (10) mit einem flexiblen Träger (12), wobei eine Anzahl von Elektroden (11) rasterförmig auf derselben Seite des Trägers (12) angeordnet sind und wobei vom äußeren Rand des Trägers (12) her Schlitze zwischen den einzelnen Elektroden (11) angeordnet sind, und
- eine elastische Zwischenschicht (20), die für jede der Elektroden (11) jeweils eine Ausnehmung (21) aufweist, wobei durch jede Ausnehmung (21) jeweils ein Hohlraum (210) zur Verfüllung mit elektrisch leitfähigem Gel vorgegeben ist,
**gekennzeichnet durch**
eine zwischen der elastischen Zwischenschicht (20) und den Elektroden (11) des Elektroden-Arrays (10) angeordnete Rückhalteschicht,
- wobei die Rückhalteschicht eine kleinere Querschnittsfläche aufweist, als die elastische Zwischenschicht (20), und
- wobei die Rückhalteschicht Kontaktöffnungen aufweist,
wobei die Kontaktöffnungen eine kleinere Querschnittsfläche aufweisen, als die Ausnehmungen (21) der Zwischenschicht (20) und
wobei die Kontaktöffnungen, insbesondere in einem an der Körperoberfläche eines Probanden (1) angeordneten Zustand der Elektrodenanordnung (100), derart mit den Ausnehmungen (21) korrespondieren, dass eine elektrisch leitfähige Verbindung zwischen den Elektroden (11) und den Hohlräumen (210), insbesondere zwischen den Elektroden (11) und einem in den Hohlräumen (210) vorhandenem elektrisch leitfähigen Gel, herstellbar ist.

2. Elektrodenanordnung (100) nach Anspruch 1, **dadurch gekennzeichnet, dass** die elastische Zwischenschicht (20) eine erste klebende Oberfläche (22) zur Anordnung an der Körperoberfläche eines Probanden (1) und/oder eine zweite klebende Oberfläche (23) zur Anordnung am Träger (12) des Elektroden-Arrays (10) aufweist.

3. Elektrodenanordnung (100) nach Anspruch 1 oder 2, **dadurch gekennzeichnet, dass** die elastische Zwischenschicht (20) aus einem flexiblen elektrisch isolierenden Material, insbesondere einem geschäumten Kunststoff, gebildet ist.

4. Elektrodenanordnung (100) nach einem der vorangehenden Ansprüche, **dadurch gekennzeichnet, dass** die Elektroden (11) des Elektroden-Arrays (10) hochleitfähig sind, und insbesondere aus Gold, Platin, elektrisch leitfähigem Polymer oder einem anderen Elektrodenmaterial bestehen.

5. Elektrodenanordnung (100) nach einem der vorangehenden Ansprüche, **dadurch gekennzeichnet, dass** der Träger (12) des Elektroden-Arrays (10) als flexible Leiterplatte, insbesondere als FlexPrint, ausgebildet ist.

6. Elektrodenanordnung (100) nach einem der vorangehenden Ansprüche, **dadurch gekennzeichnet, dass** das Elektroden-Array (10) zur Anordnung an einem Fuß und/oder am Brustkorb und/oder am Herzen ausgebildet ist.

7. Elektrodenanordnung (100) nach einem der vorangehenden Ansprüche, **dadurch gekennzeichnet, dass** die Schlitze (13) zwischen den einzelnen Elektroden (11) des Elektroden-Arrays (10) derart ausgebildet sind, dass das Elektroden-Array (10) an einer gekrümmten Körperoberfläche, insbesondere am Kopf eines Probanden (1), anordenbar ist.

8. Elektrodenanordnung (100) nach einem der vorangehenden Ansprüche, **gekennzeichnet durch** eine auf dem Träger (10) angeordnete Verstärkeranordnung (30), wobei die Elektroden (11) an die Verstärkeranordnung (30) angeschlossen sind, und wobei insbesondere vorgesehen ist, dass die Verstärkeranordnung (30) in einem Abstand von kleiner als 100 mm, insbesondere im Bereich unter 10 mm zur nächstgelegenen Elektrode (11) des Elektroden-Arrays (10) angeordnet ist.

9. Elektrodenanordnung (100) nach einem der vorangehenden Ansprüche, **gekennzeichnet durch** eine mit dem Elektroden-Array (10) verbundene Switching-Unit, wobei die Switching-Unit dazu ausgebildet ist, zwischen einer Elektro-Stimulation des menschlichen Körpers über die Elektroden (11) und einer Messung von elektrischen Spannungen und Strömen im menschlichen Körper mittels der Elektroden (11) umzuschalten.

10. Elektrodenanordnung (100) nach einem der vorangehenden Ansprüche, **dadurch gekennzeichnet, dass** die Hohlräume (210), insbesondere in einem an der Körperoberfläche eines Probanden (1) angeordneten Zustand der Elektrodenanordnung (100), mit elektrisch leitfähigem Gel gefüllt sind.

11. Elektrodenanordnung (100) nach einem der vorangehenden Ansprüche, **dadurch gekennzeichnet, dass** die elastische Zwischenschicht (20), insbesondere in einem an der Körperoberfläche eines Probanden (1) angeordneten Zustand der Elektrodenanordnung (100), vom Träger (12) des Elektroden-Arrays (10) abgedeckt ist.

## Claims

1. Electrode arrangement (100) for measuring electric voltages and currents in the human body and for electro-simulation of the human body, comprising
- an electrode array (10) with a flexible carrier (12), wherein a number of electrodes (11) are arranged in a grid on the same side of the carrier (12) and wherein slots are arranged between the individual electrodes (11) starting from the outer edge of the carrier (12), and
- an elastic intermediate layer (20) that has one recess (21) for each electrode (11), wherein each recess (21) defines a cavity (210) to be filled with an electrically conductive gel,
**characterised by**
a retaining layer arranged between the elastic intermediate layer (20) and the electrodes (11) of the electrode array (10),
- wherein the retaining layer has a smaller cross-sectional area than the intermediate elastic layer (20), and
- wherein the retention layer has contact openings,
wherein the contact openings have a smaller cross-sectional area than the recesses (21) of the intermediate layer (20) and
wherein the contact openings, in particular when the electrode arrangement (100) is arranged on the body surface of a test subject (1), correspond to the recesses (21) in such a way that an electrically conductive connection can be produced between the electrodes (11) and the cavities (210), in particular between the electrodes (11) and an electrically conductive gel present in the cavities (210).

2. Electrode arrangement (100) according to claim 1, **characterised in that** the elastic intermediate layer (20) has a first adhesive surface (22) for arrangement on the body surface of a test subject (1) and/or a second adhesive surface (23) for arrangement on the carrier (12) of the electrode array (10).

3. Electrode arrangement (100) according to claim 1 or 2, **characterised in that** the elastic intermediate layer (20) is formed from a flexible electrically insulating material, in particular a foamed plastic.

4. Electrode arrangement (100) according to any one of the preceding claims, **characterised in that** the electrodes (11) of the electrode array (10) are highly conductive, and in particular consist of gold, platinum, electrically conductive polymer or another electrode material.

5. Electrode arrangement (100) according to any one of the preceding claims, **characterised in that** the carrier (12) of the electrode array (10) is formed as a flexible printed circuit board, in particular as a flex print.

6. Electrode arrangement (100) according to any one of the preceding claims, **characterised in that** the electrode array (10) is designed to be arranged on a foot and/or on the chest and/or on the heart.

7. Electrode arrangement (100) according to any one of the preceding claims, **characterised in that** the slots (13) between the individual electrodes (11) of the electrode array (10) are formed in such a way that the electrode array (10) can be arranged on a curved body surface, in particular on the head of a test subject (1).

8. Electrode arrangement (100) according to any one of the preceding claims, **characterised by** an amplifier arrangement (30) arranged on the carrier (10), wherein the electrodes (11) are connected to the amplifier arrangement (30), and wherein it is provided in particular that the amplifier arrangement (30) is arranged at a distance of less than 100 mm, in particular in the range below 10 mm, from the nearest electrode (11) of the electrode array (10).

9. Electrode arrangement (100) according to any one of the preceding claims, **characterised by** a switching unit connected to the electrode array (10), wherein the switching unit is designed to switch between electro-stimulation of the human body via the electrodes (11) and measurement of electrical voltages and currents in the human body by means of the electrodes (11).

10. Electrode arrangement (100) according to any one of the preceding claims, **characterised in that** the cavities (210), in particular when the electrode arrangement (100) is arranged on the body surface of a test subject (1), are filled with electrically conductive gel.

11. Electrode arrangement (100) according to any one of the preceding claims, **characterised in that** the elastic intermediate layer (20), in particular when the electrode arrangement (100) is arranged on the body surface of a subject (1), is covered by the carrier (12) of the electrode array (10).

## Revendications

1. Ensemble d'électrodes (100) pour la mesure de tensions et courants électriques dans le corps humain, ainsi que pour l'électrostimulation du corps humain comprenant
- un réseau d'électrodes (10) avec un support flexible (12), dans lequel un certain nombre d'électrodes (11) sont disposés en forme de grille du même côté du support (12) et dans lequel des fentes sont disposées entre les électrodes individuelles (11) à partir du bord extérieur du support (12), et
- une couche intermédiaire élastique (20), qui présente pour chacune des électrodes (11) respectivement un évidement (21), dans laquelle respectivement un espace creux (210) est prédéfini à travers chaque évidement (21) pour le remplissage avec un gel électroconducteur,
**caractérisé par**
une couche de rétention disposée entre la couche intermédiaire élastique (20) et les électrodes (11) du réseau d'électrodes (10),
- dans lequel la couche de rétention présente une section transversale plus petite que celle de la couche intermédiaire élastique (20), et
- dans lequel la couche de rétention présente des ouvertures de contact,
dans lequel les ouvertures de contact présentent une section transversale plus petite que celle des évidements (21) de la couche intermédiaire (20) et
dans lequel les ouvertures de contact, en particulier dans un état de l'ensemble d'électrodes (100) disposé sur la surface corporelle d'un sujet (1), correspondent aux évidements (21) de telle manière qu'une liaison électroconductrice peut être établie entre les électrodes (11) et les espaces creux (210), en particulier entre les électrodes (11) et un gel électroconducteur présent dans les espaces creux (210).

2. Ensemble d'électrodes (100) selon la revendication 1, **caractérisé en ce que** la couche intermédiaire élastique (20) présente une première surface adhésive (22) pour l'agencement sur la surface corporelle d'un sujet (1) et/ou une seconde surface adhésive (23) pour l'agencement sur le support (12) du réseau d'électrodes (10).

3. Ensemble d'électrodes (100) selon la revendication 1 ou 2, **caractérisé en ce que** la couche intermédiaire élastique (20) est formée à partir d'un matériau flexible électriquement isolant, en particulier une matière plastique expansée.

4. Ensemble d'électrodes (100) selon l'une quelconque des revendications précédentes, **caractérisé en ce que** les électrodes (11) du réseau d'électrodes (10) sont de haute conductivité, et en particulier se composent d'or, de platine, d'un polymère électroconducteur ou d'un autre matériau d'électrodes.

5. Ensemble d'électrodes (100) selon l'une quelconque des revendications précédentes, **caractérisé en ce que** le support (12) du réseau d'électrodes (10) est conçu en tant que circuit imprimé flexible, en particulier en tant que FlexPrint.

6. Ensemble d'électrodes (100) selon l'une quelconque des revendications précédentes, **caractérisé en ce que** le réseau d'électrodes (10) est conçu pour être agencé sur un pied et/ou la cage thoracique et/ou le coeur.

7. Ensemble d'électrodes (100) selon l'une quelconque des revendications précédentes, **caractérisé en ce que** les fentes (13) sont conçues entre les électrodes individuelles (11) du réseau d'électrodes (10) de telle manière que le réseau d'électrodes (10) peut être disposé sur une surface corporelle recourbée, en particulier sur la tête d'un sujet (1).

8. Ensemble d'électrodes (100) selon l'une quelconque des revendications précédentes, **caractérisé par** un ensemble amplificateur disposé sur le support (10), dans lequel les électrodes (11) sont raccordées sur l'ensemble amplificateur (30), et dans lequel est prévu en particulier que l'ensemble amplificateur (30) est disposé à une distance inférieure à 100 mm, en particulier dans la zone inférieure à 10 mm par rapport à l'électrode la plus proche (11) du réseau d'électrodes (10).

9. Ensemble d'électrodes (100) selon l'une quelconque des revendications précédentes, **caractérisé par** une unité de commutation reliée au réseau d'électrodes (10), dans lequel l'unité de commutation est conçue pour basculer entre une électrostimulation du corps humain par le biais des électrodes (11) et une mesure de tensions et courants électriques dans le corps humain à l'aide des électrodes (11).

10. Ensemble d'électrodes (100) selon l'une quelconque des revendications précédentes, **caractérisé en ce que** les espaces creux (210), en particulier dans un état de l'ensemble d'électrodes (100) disposé sur la surface corporelle d'un sujet (1), sont remplis d'un gel électroconducteur.

11. Ensemble d'électrodes (100) selon l'une quelconque des revendications précédentes, **caractérisé en ce que** la couche intermédiaire élastique (20), en particulier dans un état de l'ensemble d'électrodes (100) disposé sur la surface corporelle d'un sujet (1), est recouverte par le support (12) du réseau d'électrodes (10).
